(19) Europäisches Patentamt
European Patent Office
Office européen des brevets

(11) EP 2 397 164 B1

(12) **EUROPEAN PATENT SPECIFICATION**

(45) Date of publication and mention of the grant of the patent:
**08.04.2015 Bulletin 2015/15**

(51) Int Cl.:
*A61L 27/52* (2006.01) *A61L 27/58* (2006.01)

(21) Application number: **11007569.4**

(22) Date of filing: **13.02.2008**

(54) **Low-swelling biocompatible hydrogels**

Schwachquellende biokompatible Hydrogele

Hydrogels biocompatibles à faible gonflement

(84) Designated Contracting States:
**DE ES FR GB IE IT**

(30) Priority: **05.03.2007 US 714028**

(43) Date of publication of application:
**21.12.2011 Bulletin 2011/51**

(62) Document number(s) of the earlier application(s) in accordance with Art. 76 EPC:
**08250526.4 / 1 967 220**

(73) Proprietor: **Confluent Surgical Inc.**
**Waltham, Massachusetts 02451 (US)**

(72) Inventors:
- **Sawhney, Amarpreet S.**
**Lexington, MA 02421 (US)**
- **Bennett, Steven L.**
**Cheshire, CT 06410 (US)**

(74) Representative: **Soames, Candida Jane et al**
**Maschio & Soames IP Limited**
**20 Carlton Crescent**
**Southampton, SO15 2ET (GB)**

(56) References cited:
**EP-A- 1 704 878**
**US-A1- 2003 012 734**
**US-B1- 6 818 018**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 397 164 B1

## Description

The present disclosure is related to covalently-crosslinked hydrogels for contact with spinal dura mater.

Hydrogels may be used in the body for applications such as sealing, adhesion prevention, or drug delivery. Hydrogels can exhibit a generally high degree of swelling when hydrated.

Certain medical applications, however, do not tolerate a high degree of swelling. For instance, GELFOAM® absorbable gelatin (Pharmacia & Upjohn, Kalamazoo, MI) is a water-insoluble, porous, pliable form of gelatin for application to bleeding surfaces as a hemostatic. However, GELFOAM® is not suited for applications around the vertebral column and is contra-indicated for laminectomy procedures and for use near foramina in bone, once hemostasis is achieved. This contraindication exists because GELFOAM® may swell after absorbing physiological fluids and produce nerve damage by pressure within confined bony spaces. The packing of GELFOAM®, particularly within bony cavities, should be avoided, since swelling may interfere with normal function and/or possibly result in compression necrosis of surrounding tissues

US 2003/012734 discloses a composition comprising a cross linking hydrogel, for use as an adhesion barrier around the spinal cord, including first and second pre-cursers.

## SUMMARY

These concerns, however, are addressed by low-swelling hydrogels according to the composition of claim 1.

The first functional groups crosslink with the second functional groups thereby forming a hydrogel which swells from -50% to 50%.

In embodiments, the hydrogel may be crosslinked to form a gel from a first synthetic precursor possessing first functional groups and a second synthetic precursor possessing second functional groups in less than about 10 seconds after mixing the first precursor and second precursor with each other. In some embodiments, the first synthetic precursor may be dilysines, trilysines, tetralysines, or an oligopeptide sequence of no more than five residues possessing at least two lysine groups.

The present disclosure describes a method for treating tissue inside a vertebral column by forming a low-swelling biodegradable hydrogel in situ adherent to tissue inside the vertebral column and substantially exterior to a theca in the vertebral column.

The hydrogel may be applied to a location within a patient such that the hydrogel is located in a peridural or an epidural space. The surrounding tissue may include, for example, a spinal nerve or spinal nerve root exterior to the theca. The hydrogel may be crosslinked to form a firm gel from a first precursor and a second precursor in less than about 10 seconds after mixing the first precursor and second precursor with each other.

The present disclosure describes kits for producing hydrogels which include a first synthetic precursor possessing first functional groups, and a second synthetic precursor including a multi-armed precursor possessing a core and from about 3 to about 12 arms, the arms each including a polyethylene glycol having a molecular weight from about 250 to about 5000 and possessing second functional groups at the ends thereof. The first functional groups crosslink with the second functional groups thereby forming a hydrogel which swells from about -50% to about 50%.

## BRIEF DESCRIPTION OF THE FIGURES

Figure 1 is a side view illustration of a portion of a vertebral column; and
Figure 2 is a top, cross-sectional view of an embodiment of a hydrogel coating disposed in a vertebral column.

## DETAILED DESCRIPTION OF PREFERRED EMBODIMENTS

Hydrogels are described herein that may be suitable for use in a vertebral column area. These hydrogels have good adhesion to tissues, can be formed in-situ, are biodegradable, and exhibit low-swelling after placement so that soft tissues will not be unduly compressed against bony portions of the vertebral column. Nerves, in particular, may be vulnerable when a conventional hydrogel swells and compresses a nerve against a bone. Thus, low-swelling hydrogel compositions described herein may create new therapeutic possibilities for treating tissues around nerves in bony areas.

Hydrogels can be useful aids in surgical procedures for use, for example, as hemostats, sealants, protective barriers, and the like. The creation of hydrogels in situ in a patient may enable the creation of a hydrogel that coats tissue, conforms to its shape, and fills/conforms to a three dimensional space. Such materials should possess mechanical properties adequate to withstand strains caused by movement of the patient, shifting of tissues, hydrostatic forces present in the tissue, and the like. At the same time, a high water content can be useful for biocompatibility.

*Hydrogel Systems Overview*

Certain hydrogel properties can be useful, such as adhesion to a variety of tissues, fast setting times to enable a surgeon to accurately and conveniently place the hydrogels, high water content for biocompatibility, mechanical strength for use in sealants, and/or toughness to resist destruction after placement. Synthetic materials that are readily sterilized and avoid the dangers of disease transmission involved in the use of natural materials may thus be used. Indeed, certain in situ polymerizable hydrogels made using synthetic precursors are within the purview of those skilled in the art, e.g., as used in commercially available products such as FOCALSEAL® (Genzyme, Inc.), COSEAL® (Angiotech Pharmaceuticals), and DURASEAL® (Confluent Surgical, Inc). Other known hydrogels include, for example, those disclosed in U.S. Patent Nos. 6,656,200; 5,874,500; 5,543,441; 5,514,379; 5,410,016; 5,162,430; 5,324,775; 5,752,974; and 5,550,187.

The swelling of COSEAL® and DURASEAL® has been measured using an in vitro model in comparison to fibrin sealant (Campbell et al., Evaluation of Absorbable Surgical Sealants: In vitro Testing, 2005). Over a three day test, COSEAL® swelled an average of about 558% by weight, DURASEAL® increased an average of about 98% by weight, and fibrin sealant swelled by about 3%. Assuming uniform expansion along all axes, the percent increase in a single axis was calculated to be 87%, 26%, and 1% for COSEAL®, DURASEAL®, and fibrin sealant respectively. Hydrogels with less swelling would be desirable for applications at or near the vertebral column. Fibrin sealant is a proteinaceous glue that has adhesive, sealing, and mechanical properties that are inferior to COSEAL®, DURASEAL®, and other hydrogels disclosed herein. Further, it is typically derived from biological sources that are potentially contaminated, is cleared from the body by mechanisms distinct from this class of hydrogels, and typically requires refrigeration while stored.

In situ polymerizable hydrogels may be made from precursors. The precursor may be, e.g., a monomer or a macromer. One type of precursor may have a functional group that is ethylenically unsaturated. An ethylenically unsaturated functional group may be polymerized using an initiator to start the reaction. Precursors with at least two ethylenically unsaturated functional groups may form crosslinked polymers. Some compositions have certain precursors with only one such functional group and additional crosslinker precursors with a plurality of functional groups for crosslinking the precursors. Ethylenically unsaturated functional groups may be polymerized by various techniques, e.g., free radical, condensation, or addition polymerization. Hydrogels may be formed from one precursor (as by free radical polymerization), two precursors, or made with three or more precursors, with one or more of the precursors participating in crosslinking to form the hydrogel.

Another type of precursor has a functional group that is an electrophile or nucleophile. Electrophiles react with nucleophiles to form covalent bonds. Covalent crosslinks or bonds refer to chemical groups formed by reaction of functional groups on different polymers that serve to covalently bind the different polymers to each other. In certain embodiments, a first set of electrophilic functional groups on a first precursor may react with a second set of nucleophilic functional groups on a second precursor. When the precursors are mixed in an environment that permits reaction (e.g., as relating to pH or solvent), the functional groups react with each other to form covalent bonds and join the precursors together. The precursors become crosslinked when at least some of the precursors can react with more than one other precursor. For instance, a precursor with two functional groups of a first type may be reacted with a crosslinking precursor that has at least three functional groups of a second type capable of reacting with the first type of functional groups.

*Hydrogels and Precursor Materials*

Suitable hydrogels for use in accordance with the present disclosure include macromolecular and polymeric materials into which water and small hydrophilic molecules can easily diffuse. Hydrogels of interest include, e.g., those prepared through the cross-linking of: polyethers, e.g. polyakyleneoxides such as poly(ethylene glycol), poly(ethylene oxide), poly(ethylene oxide)-co-(poly(propyleneoxide) block copolymers; poly(vinyl alcohol); and poly(vinyl pyrrolidone). Because of their high degree of biocompatibility and resistance to protein adsorption, polyether derived hydrogels may be useful in some embodiments, including poly(ethylene glycol) derived hydrogels.

Natural polymers, for example proteins, polysaccharides, or glycosaminoglycans, may also be used, as well as derivatives thereof, e.g., hyaluronic acid, dextran, chondroitin sulfate, heparin, heparin sulfate, alginate, gelatin, collagen, albumin, ovalbumin, polyamino acids, collagen, fibrinogen, albumin, fibrin, starch, dermatan sulfate, keratan sulfate, dextran sulfate, pentosan polysulfate, chitosan, fibronectin, laminin, elastin, and active peptide domains thereof. Such polymers may be reacted via functional groups such as amines, thiols, or carboxyls on their amino acids, or derivatized to have activatable functional groups. While natural polymers may be used in low-swelling hydrogels, their time to gelation and ultimate mechanical properties should be controlled by appropriate introduction of additional functional groups and selection of suitable reaction conditions, e.g., pH. For example, fibrin glues, which rely on polymerization of fibrinogen to form fibrin, have a limited range of mechanical properties, a limited range of degradability, and thus may not be suitable to all of the therapeutic applications that are available when low-swelling hydrogels as described herein are formulated. However, it is contemplated such natural materials may be utilized in some embodiments.

The precursors may have biocompatible and water soluble core groups. As used herein, water soluble refers

to a solubility of at least about 1 g/l in water. This core group is a water soluble molecule with a minimum of three arms. An arm on a hydrogel precursor refers to a linear chain of chemical groups that connect a crosslinkable functional group to a multifunctional center which initiates the polymerization of the polymeric arms. The combination of this multifunctional center and the attached arms comprises the core group. A crosslinkable functional group on a hydrogel precursor arm is a chemical group that participates in a covalent crosslinking reaction between two hydrogel precursor arms. In embodiments, the core group may be a water soluble polymer. Examples of such polymers that may be used include, for example: polyethers, for example, polyalkylene oxides such as polyethylene glycol ("PEG"), polyethylene oxide ("PEO"), polyethylene oxide-co-polypropylene oxide ("PPO"), co-polyethylene oxide block or random copolymers; polyvinyl alcohol ("PVA"); poly (vinyl pyrrolidinone) ("PVP"); poly (amino acids); dextran; and proteins, as well as derivatives of the foregoing and combinations of the foregoing.

In other embodiments, multifunctional centers may include polyols which, in embodiments, may possess hydroxyl groups for initiation of monomeric groups that form the arms of the core that can then be functionalized with crosslinkable groups. Depending on the desired number of arms, the polyol may possess from about 3 to about 12 hydroxyl groups, from about 5 to about 10 hydroxyl groups. The polyol may also possess other protected or unprotected functional groups. Suitable polyols include glycerol, mannitol, reducing sugars such as sorbitol, pentaerythritol, and glycerol oligomers including hexaglycerol, as well as derivatives thereof and combinations thereof. As would be readily apparent to one skilled in the art, the number of hydroxyl groups should be equivalent to the number of arms on the multi-armed precursor, i.e., the particular polyol chosen will determine the number of arms on the resultant multifunctional core group. In embodiments, a polymer described above, such as polyethylene glycol, would be formed by initiating the polymerization of ethylene oxide with the polyol thereby forming arms of a multi-armed precursor that may be further functionalized.

Thus hydrogels can be made from a multi-armed precursor with a first set of functional groups and a low molecular weight precursor having a second set of functional groups.

For example, a multi-armed precursor may have hydrophilic arms, e.g., polyethylene glycol, terminated with N-hydroxy succinimide, with the combined molecular weight of the arms being from about 1,000 to about 40,000; artisans will immediately appreciate that all ranges and values within the explicitly stated bounds are contemplated. The invention utilizes a multi-armed precursor having six arms or eight arms. The molecular weight of an individual arm of such a precursor is from 1250 to 2500.

In some embodiments, six-armed or eight-armed precursors may be reacted with a low molecular weight precursor such as trilysine. The trilysine provides multiple points of reaction for crosslinking the multi-armed precursors and it presumably (without being limited to a particular theory of action) allows relatively little movement in terms of shrinking or swelling, with such movements probably being related to the multi-armed precursors, which are relatively larger and more mobile. Accordingly, other small molecules may be used instead of trilysine, for example, molecules with a molecular weight of from about 100 to about 5000, in embodiments from about 300 to about 2500, in other embodiments from about 500 to about 1500. Such small molecules may have at least about three functional groups, from about 3 to about 16 functional groups; ordinary artisans will appreciate that all ranges and values between these explicitly articulated values are contemplated. Such small molecules may be polymers or non-polymers, and may be natural or synthetic. In some cases dilysines and/or tetralysines may also be utilized as the low molecular weight precursor.

Synthetic refers to a molecule not found in nature and does not include a derivatized version of a natural biomolecule, e.g., collagen with modified side groups. Polyamino acid polymers generated synthetically are normally considered to be synthetic if they are not found in nature and are engineered to not be identical to naturally occurring biomolecules. For instance, trilysine is synthetic since it is not found in nature (even though some bacteria might produce relatively larger polylysines).

Some embodiments include a precursor that includes an oligopeptide sequence of no more than about five residues having at least two lysine groups. A residue is an amino acid, either as occurring in nature or derivatized thereof. The backbone of such an oligopeptide may be natural or synthetic. In some embodiments, two or more lysines may be combined with a synthetic backbone to make a precursor; certain embodiments of such precursors may have a molecular weight from about 100 to about 10,000, in embodiments from about 300 to about 5000; artisans will immediately appreciate that all ranges and values between these explicitly articulated bounds are contemplated.

Some hydrogels may be made with a polyethylene glycol-containing precursor. Polyethylene glycol (PEG, also referred to herein as polyethylene oxide) refers to a polymer with a repeat group $(CH_2CH_2O)_n$, with n being at least 3. A polymeric precursor having a polyethylene glycol thus may have at least three of these repeat groups connected to each other in a linear series. The polyethylene glycol content of a polymer or arm may be calculated by adding up all of the polyethylene glycol groups on the polymer or arm, even if they are interrupted by other groups. Thus, an arm having at least 1000 MW polyethylene glycol has enough $CH_2CH_2O$ groups to total at least 1000 MW. As is customary terminology in these arts, a polyethylene glycol polymer does not necessarily terminate in a hydroxyl group.

In certain embodiments, precursors may include compositions that are biodegradable, crosslinkable, and substantially water soluble macromers. The macromers may possess at least one water soluble region, at least one degradable regions, and the arms of such a precursor may possess statistically more than 1 polymerizable region on average, so that a three-armed precursor would possess at least three polymerizable regions. The polymerizable regions may be separated from each other by at least one degradable region. Alternatively, if biodegradability is not desirable, compositions that do not contain the biodegradable segments, but may be water soluble and crosslink in vivo under acceptable physiological conditions, may be used.

A monomeric or macromeric precursor capable of being crosslinked to form a biocompatible material may be used to form the hydrogels. These may be small molecules, such as acrylic acid or vinyl caprolactam, larger molecules containing polymerizable groups, such as acrylate-capped polyethylene glycol (PEG-diacrylate), or other polymers containing ethylenically-unsaturated groups, such as those of U.S. Patent No. 4,938,763 to Dunn et al., U.S. Patent Nos. 5,100;992 and 4,826,945 to Cohn et al., or U.S. Patent Nos. 4,741,872 and 5,160,745 to De Luca et al..

Suitable macromeric precursors which may be utilized include the crosslinkable, biodegradable, water-soluble macromers described in U.S. Patent No. 5,410,016 to Hubbell et al. These monomers may be characterized by having at least two polymerizable groups, separated by at least one degradable region. When polymerized in water, these monomers may form coherent gels that persist until eliminated by self-degradation. The macromers are self-condensible, meaning that they may react with each other and not with proteins or other moieties on nearby tissues.

Precursors with longer distances between crosslinks are generally softer, more compliant, and more elastic. Thus, an increased length of a water-soluble segment, such as a polyethylene glycol, may enhance elasticity to produce desirable physical properties. Thus the present disclosure may be directed to precursors with water soluble segments having molecular weights from about 3,000 to about 100,000, and about 10,000 to about 35,000.

*Biodegradable Linkages*

As noted above, one or more precursors having biodegradable linkages present in between functional groups are used to make the hydrogel biodegradable or absorbable. In some embodiments, these linkages may be, for example, esters, which may be hydrolytically degraded in physiological solution. The use of such linkages is in contrast to protein linkages that may be degraded by proteolytic action. A biodegradable linkage optionally also may form a part of a water soluble core of one or more of the precursors. Alternatively, or in addition, functional groups of precursors may be chosen such that the product of the reaction between them results in a biodegradable linkage. For each approach, biodegradable linkages may be chosen such that the resulting biodegradable biocompatible crosslinked polymer degrades or is absorbed in a desired period of time. Generally, biodegradable linkages may be selected that degrade the hydrogel under physiological conditions into non-toxic or low toxicity products.

The biodegradable linkage may be chemically or enzymatically hydrolyzable or absorbable. Illustrative chemically hydrolyzable biodegradable linkages include polymers, copolymers and oligomers of glycolide, di-lactide, 1-lactide, caprolactorie, dioxanone, and trimethylene carbonate. Other chemically hydrolyzable biodegradable linkages can be monomeric in form, for example those formed through ring opening of glutaric anhydride with poly(ethylene glycol) to form a glutaric acid linkage. Other linkages include succinic, maleic, methyl succinic, diglycolic methyl glutaric, combinations thereof, and the like. Illustrative enzymatically hydrolyzable biodegradable linkages include peptidic linkages cleavable by metalloproteinases and collagenases. Additional illustrative biodegradable linkages include polymers and copolymers of poly(hydroxy acid)s, poly(orthocarbonate)s, poly(anhydride)s, poly(lactone)s, and poly(phosphonate)s.

Natural polymers may be proteolytically degraded by proteases present in the body, which are enzymes that recognize specific biological moieties such as amino acid sequences. In contrast, synthetic polymers without such specifically cleavable sequences may be degraded by other mechanisms such as hydrolytic degradation. In the spinal cord, synthetic polymers free of such sequences can be expected to undergo little or no degradation by specific enzymatic action. Nonspecific attack and degradation by nonspecifically acting enzymes may result in a different biological response and time to degradation and is not equivalent to degradation by enzymes that are specific to a particular amino acid sequence. Some include precursors that do not have sequences subject to specific recognition and cleavage by enzymes.

*Functional Groups*

Functional groups on the precursors include chemical moieties that react with other functional groups to form a covalent bond as part of the process of making a hydrogel. Functional groups include, for example, ethylenically unsaturated polymerizable groups, e.g., pendent vinyl groups, acrylate groups, methacrylate groups, ethacrylate groups, 2-phenyl acrylate groups, acrylamide groups, methacrylamide groups, itaconate groups, styrene groups, combinations thereof, and the like.

Functional groups can include electrophilic or nucleophilic groups that participate in an electrophilic-nucleophilic reaction to form a hydrogel. Examples of electrophilic functional groups include carbodiimidazole groups, sulfonyl chloride groups, chlorocarbonate groups, n-hydroxysuccinimidyl ester groups, succinimidyl ester groups, sulfasuccinimidyl ester groups, N-hydroxyethoxylated succinimide ester groups, methane diisocyanate groups, methylene-bis(4-cyclohexylisocyanate) groups, isocyanate groups, diisocyanate groups, hexamethylenediisocyanate groups, maleimide groups, and the like. Examples of nucleophilic functional groups include amine groups, hydroxyl groups, carboxyl groups, thiol groups, and the like.

*Initiating Systems*

An initiator group is a chemical group capable of initiating a free radical polymerization reaction. For instance, it may be present as a separate component, or as a pendent group on a precursor. Initiator groups include thermal initiators, photoactivatable initiators, oxidation-reduction (redox) systems, combinations thereof, and the like.

Long wave UV and visible light photoactivatable initiators include, for example, ethyl eosin groups, 2,2-dimethoxy-2-phenyl acetophenone groups, other acetophenone derivatives, thioxanthone groups, benzophenone groups, camphorquinone groups, combinations thereof, and the like.

Examples of thermally reactive initiators include 4,4' azobis(4-cyanopentanoic acid) groups, analogs of benzoyl peroxide groups, combinations thereof, and the like. Several commercially available low temperature free radical initiators, such as V-044, available from Wako Chemicals USA, Inc. (Richmond, VA) may be used to initiate free radical crosslinking reactions at body temperatures to form hydrogels with the aforementioned monomers.

Metal ions may also be used either as an oxidizer or a reductant in redox initiating systems. For example, ferrous ions may be used in combination with a peroxide or hydroperoxide to initiate polymerization, or as parts of a polymerization system. In this case, the ferrous ions would serve as a reductant. Alternatively, metal ions may serve as an oxidant For example, the ceric ion (4+ valence state of cerium) may interact with various organic groups, including carboxylic acids and urethanes, to remove an electron to the metal ion, thus leaving an initiating radical behind on the organic group. In such a system, the metal ion acts as an oxidizer. Potentially suitable metal ions for either role are any of the transition metal ions, lanthanides and actinides, which have at least two readily accessible oxidation states. Metal ions may have at least two states separated by only one difference in charge. Of these, the most commonly used include ferric/ferrous; cupric/cuprous; ceric/cerous; cobaltic/cobaltous; vanadate V vs. IV; permanganate; and manganic/manganous. Peroxygen containing compounds, such as peroxides and hydroperoxides, including hydrogen peroxide, t-butyl hydroperoxide, t-butyl peroxide, benzoyl peroxide, and cumyl peroxide, may also be used.

An example of an initiating system is the combination of a peroxygen compound in one solution, and a reactive ion, such as a transition metal, in another. In this case, no external initiators of polymerization may be needed and polymerization may proceed spontaneously and without application of external energy or use of an external energy source when two complementary reactive functional groups containing moieties interact at the application site.

*Hydrogel Swellability*

It has been found that changing the length of the arms on a precursor while holding other properties generally constant can alter the swelling properties of the resultant gel from one that swells to one that shrinks. At any given concentration of reactive polymer, an arm length can be utilized that provides for a low-swelling gel with minimal compromise of other properties. Without being bound to a particular theory, changing the arm length can approximate the distance between crosslinks at equilibrium swelling. The closer the arm length is to equilibrium crosslink distance, the less the arms extend in response to swelling. As described herein, hydrogels may be made in situ in a patient with a low, or even negative, amount of swelling. Such hydrogels may be formulated with mechanical properties for adhesion and/or sealing. In contrast, conventional hydrogels for in situ polymerization that have mechanical properties for adhesion and/or sealing lack low-swelling properties and are not suited for use in a vertebral column.

Thus, desirable hydrogels described herein can include low-swelling hydrogels with a reaction time, density, strength, and desirable medical properties that are made using components selected from a class of precursors in a desirable molecular-weight range, solubility, arm-length, chemical composition, chemical structure, chemical composition, density, precursor concentration, arm number, and with desired functional groups and buffers. Some of these parameters are interrelated so that the choice of one range of starting properties or materials can affect the choice of other properties and materials.

Unless otherwise indicated, swelling of a hydrogel relates to its change in volume (or weight) between the time of its formation when crosslinking is effectively complete and the time after being placed in a physiological solution in an unconstrained state for twenty-four hours, at which point it may be reasonably assumed to have achieved its equilibrium swelling state. For most embodiments, crosslinking is effectively complete within no more than about fifteen minutes, and often within a few seconds, such that the initial weight can be reasonably noted as Weight at initial formation. Accordingly, the following formula may be used to determine swelling:

% swelling = [(Weight at 24 hours - Weight at initial formation)/ Weight at initial formation] * 100.

Low-swellable or low-swelling hydrogels of the present disclosure may have a weight upon polymerization that increases no more than, e.g., about 50% by weight upon exposure to a physiological solution, or that shrink (decrease in weight and volume), e.g., by about 5% or more. This is contrary to other hydrogels, which may experience swelling in amounts of from about 300% to about 600% by weight upon exposure to a physiological solution. For example, hydrogels thai have a weight increase from formation to equilibrium hydration of no more than from about 0% to about 50%, from about 10% to about 40%, or swell from about 0% to about 50%, from about 5% to about 40%, or shrink by a weight decrease of from about 1% to about 50%, from about 5% to about 30%. Again, swelling or shrinking is determined by the change in weight of the hydrogel upon exposure to a physiological solution utilizing the formula set forth above. Shrinkage is referred to herein as a negative % swelling; thus, a hydrogel of the present disclosure swells from about -50% to about 50%, may swell from about -20% to about 40%. Artisans will immediately appreciate that all ranges and values within or otherwise relating to these explicitly articulated limits are disclosed herein.

The weight of the hydrogel includes the weight of the solution in the hydrogel. A hydrogel formed in a location wherein it is constrained is not necessarily a low-swelling hydrogel. For instance, a swellable hydrogel created in a body may be constrained from swelling by its surroundings but nonetheless may be a highly swellable hydrogel as evidenced by measurements of its swelling when unconstrained and/or the forces against a constraint.

The solids content of the hydrogel which has crosslinked and is at equilibrium can affect its mechanical properties and biocompatibility and reflects a balance between competing requirements. In general, a relatively low solids content may be desirable, e.g., from about 5% to about 25% of the combined weight of the hydrogel in an aqueous solution, all ranges and values therebetween, e.g., from about 5% to about 10%, from about 10% to about 15%, from about 5% to about 15%, and less than about 15%, or less than about 20%.

*In situ Polymerization*

Formulations may be prepared that are suited to make precursor crosslinking reactions occur "in situ", meaning they occur at a tissue in a living animal or human body. In general, this may be accomplished by having a precursor that can be activated at the time of application to a tissue to form a crosslinked hydrogel. Activation can be made before, during, or after application of the precursor to the tissue, provided that the precursor is allowed to conform to the tissue's shape before crosslinking and associated gelation is otherwise too far advanced. Activation includes, for instance, triggering a polymerization process, initiating a free radical polymerization, or mixing precursors with functional groups that react with each other. Thus, in situ polymerization includes activation of chemical moieties to form covalent bonds to create an insoluble material, e.g., a hydrogel, at a location where the material is to be placed on, within, or both on and within, a patient. In situ polymerizable polymers may be prepared from precursors that can be reacted such that they form a polymer within the patient. Thus precursors with electrophilic functional groups can be mixed or otherwise activated in the presence of precursors with nucleophilic functional groups. In other examples, precursors with ethylenically unsaturated groups can be initiated to polymerize in situ on the tissue of a patient.

Certain functional groups, such as alcohols or carboxylic acids, do not normally react with other functional groups, such as amines, under physiological conditions (e.g., pH 7.2, 37°C). However, such functional groups can be made more reactive by using an activating group such as N-hydroxysuccinimido. Suitable activating groups include carbonyldiimidazole, sulfonyl chloride, aryl halides, sulfosuccinimidyl esters, N-hydroxysuccinimidyl ester, succinimidyl ester, epoxide, aldehyde, maleimides, imidoesters and the like. The N-hydroxysuccinimide esters or N-hydroxysulfosuccinimide groups may be groups of particular interest for crosslinking of proteins or amine functionalized polymers such as amino terminated polyethylene glycols.

Hydrogels may be formed either through covalent, ionic or hydrophobic bonds introduced through, e.g., chemical cross-linking agents or electromagnetic radiation, such as ultraviolet light, of both natural and synthetic hydrophilic polymers, including homo and co-polymers. Physical (non-covalent) crosslinks may result from, e.g., complexation, hydrogen bonding, desolvation, Van der Waals interactions, or ionic bonding, and may be initiated by mixing components that are physically separated until combined in situ, or as a consequence of a prevalent condition in the physiological environment, such as temperature, pH, and/or ionic strength. Covalent crosslinking may be accomplished by any of a number of mechanisms, including free radical polymerization, condensation polymerization, anionic or cationic polymerization, step growth polymerization, and electrophile-nucleophile reaction.

Hydrogel systems may include those biocompatible multi-component systems that spontaneously crosslink

when the components are mixed, but wherein the two or more components are individually stable for the duration of the deposition process. Such systems include, for example, a first component including macromers that are di or multifunctional amines and a second component including di or multifunctional oxirane containing moieties. Other initiator systems, such as components of redox type initiators, may also be used.

In addition, hydrogels formed in accordance with the methods of the present disclosure may be used as coatings. Such coatings may be formed as laminates (i.e., having multiple layers). Thus, for example, a lower layer of the laminate may possess a more tightly crosslinked hydrogel that provides good adherence to the tissue surface and serves as a substrate for an overlying compliant coating to reactively bond thereto. Materials having lower molecular weights between crosslinks may be suitable for use as a base coating layer. Molecular weights from about 400 to about 20,000 of polyethylene glycol may be useful for such applications, with molecular weights from about 500 to about 10,000.

Some examples of forming a hydrogel involve mixing precursors that crosslink quickly after application to a surface, e.g., on a tissue of a patient, to form a biodegradable hydrogel. With respect to coating a tissue, and without limiting the present disclosure to a particular theory of operation, it is believed that reactive precursor species that crosslink quickly after contacting a tissue surface may form a three dimensional structure that is mechanically interlocked with the coated tissue. This interlocking contributes to adherence, intimate contact, and continuous coverage of the coated region of the tissue. The crosslinking reaction leading to gelation can occur, within a time from about 1 seconds to about 5 minutes, from about 3 seconds to about 1 minute; persons of ordinary skill in these arts will immediately appreciate that all ranges and values within these explicitly stated ranges are contemplated. In some cases gelation may occur in less than 10 seconds

The precursors may be placed into solution prior to use, with the solution being delivered to the patient. The hydrogel system solutions should not contain harmful or toxic solvents. The precursors may be substantially soluble in water to allow application in a physiologically-compatible solution, such as buffered isotonic saline. One may use a dual syringe or similar device to apply the precursor solutions, such as those described in U.S. Patent Nos. 4,874,368; 4,631,055; 4,735,616; 4,359,049; 4,978,336; 5,116,315; 4,902,281; 4,932,942; 6,179,862 ; 6,673,093; and 6,152,943. Further, such precursors may be used in combination with visualization agents such as a dye. Suitable dyes are within the purview of those skilled in the art and may include, for example, a dye for visualizing a thickness of the hydrogel as it is formed in situ, e.g., as described in U.S. Patent No. 7,009,034. In some embodiments, a suitable dye may include FD&C Blue #1, FD&C Blue #2, FD&C Blue #3, D&C Green #6, methylene blue, combinations thereof, and the like.

Hydrogels described herein include low-swelling, in-situ formed, precursor based medical crosslinked hydrogels, which optionally possess a gelation time in situ of less than about twenty seconds (or less than about ten seconds or less than about five seconds). Such hydrogels may be made with precursors having a solubility of from about 1 gram per liter to at least about 10 grams per liter. Such hydrogels may be prepared with a 1:1 ratio of reactive functional groups (e.g., electrophile: nucleophile) or other ratios as suited to the formulation. Buffers may be used to provide a pH for maintaining the activity of a reactive functional group in solution ("pot life") and to provide a desired osmotic balance when mixed, e.g., a physiological range (see U.S. Patent No. 7,009,034). The arms may have a terminal functional group, or a functional group, e.g., within no more than from about 10,000 to about 5,000 MW of the free end of an arm. At least one functional group, more than one functional group, or a combination thereof may be present. The number of arms for at least one precursor of the lowswelling hydrogel may be from about 3 to about 12, from about 5 to about 10.

An example of an 8-armed precursor, having PEG arms functionalized with succinimidyl glutrate, includes, for example, the following:

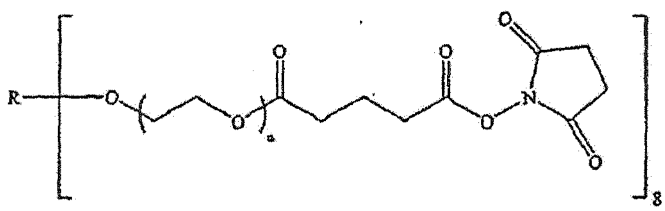

wherein R is a core as described above, a hexaglycerin core, and n may be from about 4 to about 150, from about 10 to 100. The total molecular weight of the 8 arms may be about 20,000.

*Applications at the Vertebral Column*

Nerves near the vertebral column may be vulnerable to compression in response to tissue inflammation or swelling of materials surgically placed into the body. While the body can normally tolerate a certain amount of swelling of implanted materials, swelling near a bone or rigid implant may be less tolerated because forces may be directed away from bone towards sensitive soft tissue. It thus may be desirable to avoid compressing a nerve in this manner.

The vertebral column (also referred to as the spinal column) has 33 vertebrae in humans. Each of the vertebrae is donut-shaped with an opening in the middle (the spinal canal). Positioned between almost every vertebra are the intervertebral discs. Referring to Figures 1 and 2, which depict exemplary vertebral anatomy (Figure 1 is a side view and Figure 2 is a top, cross-sectional view), an invertebral foramen 20 is defined by the shape of the vertebrae 22, 24 that lie on top of each other, with the superior notch of the inferior lamina and the inferior notch of the superior lamina forming the lower and upper borders of the intervertebral foramen. The invertebral foramen boundaries are further delineated by intervertebral disc 26 anteriorly and the pedicles of neighboring vertebrae superiorly and inferiorly. These openings allow the nerve fibers 28, 30 in the spinal cord to exit the spinal canal and travel to their specific body parts.

Spinal cord 32 is surrounded by theca 34, which is the dura mater of the spinal cord, which lies in the spinal canal. The space between theca 34 and surrounding vertebrae is epidural space 36. The space inside the spinal column and between nerves 28, 30 and vertebra 22, 24 is the peridural space 38. The portion of a nerve near its exit point from the theca is part of a spinal nerve root. Figure 2 further depicts hydrogel coating 40 using a hydrogel of the present disclosure in the epidural space that adheres to injury 42 and/or theca 34.

*Methods of Using Biocompatible Polymers*

As noted above, application for a low-swelling hydrogel of the present disclosure is for use in or around a vertebral column. The low-swelling nature of the hydrogel minimizes compression of tissues, especially nerves, against the bone. The hydrogel may be applied exterior to the theca, which is the dura mater of the spinal cord. In some applications, the hydrogel may be applied substantially exterior to a theca in the vertebral column, meaning that the hydrogel is applied in the vertebral column even while the theca is damaged or even breached, but excluding situations wherein the spinal cord is essentially severed and the hydrogel is placed into the nerve gap. The hydrogel also may contact associated vertebral-column structures, and fill some or all of the vertebral foramen, and regions outside, including nerve roots and nerve portions exterior to the theca and within, e.g.; from about 0.1 cm to about 5 cm of the vertebral column, from about 1 cm to about 4 cm of the vertebral column. As such, the hydrogel may function as a tissue adhesive, tissue sealant, drug delivery vehicle, wound covering agent, barrier to prevent postoperative adhesions, or a covering of inflamed or injured sites. The hydrogel may be applied as a bolus that fills a void or lumen and/or as a coating that conforms to a tissue surface.

The hydrogels may advantageously be used for drug delivery. Biologically active agents or drug compounds that may be added and delivered from the crosslinked polymer or gel include, for example: proteins, glycosaminoglycans, carbohydrates, nucleic acids, inorganic and organic biologically active compounds. Specific biologically active agents include but are not limited to: enzymes, antibiotics, antimicrobials, antineoplastic agents, local anesthetics, hormones, angiogenic agents, anti-angiogenic agents, growth factors, antibodies, neurotransmitters, psychoactive drugs, anticancer drugs; chemotherapeutic drugs, drugs affecting reproductive organs, genes, anti-inflammatory drugs, analgesics, antibiotics, anti-proliferatives, anti-fibrotics, and oligonucleotides.

The bioactive compounds described above may be mixed with a precursor prior to making the aqueous solution or during the aseptic manufacturing of the precursor. This mixture may then be mixed with another precursor to produce a crosslinked material in which the biologically active substance is entrapped. Precursors made from inert polymers like PLURONICS®, TETRONICS®, or TWEEN® surfactants may be used, for example, with small molecule hydrophobic drugs.

The active agent or agents may be present in a separate phase when precursors are reacted to produce a crosslinked polymer network or gel. This phase separation may prevent participation of bioactive substances in a chemical crosslinking reaction. The separate phase may also help to modulate the release kinetics of active agent from the crosslinked material or gel, where 'separate phase' could be an oil (for example, an oil-in water emulsion), a biodegradable vehicle, and the like.

EXAMPLES

Example 1: Low swelling hydrogel formulations

To prepare the hydrogels, trilysine with primary amine functional groups was reacted with multiarmed polyethylene glycol (PEG) electrophilic precursors with succinimidyl ester electrophilic functional groups (specifically, succinimidyl glutarate, SG) on the end of each of four arms (4a) having a total MW of about 20,000 MW polyethylene glycol (sometimes referred to herein as 4a20k SG) in a 1:1 stoichiometric ratio of electrophiles: nucleophiles.

Essentially identical hydrogels were then made, with the ratios of the electrophilic-nucleophilic functional groups still being 1:1, except that a 6-armed (6a) or 8-armed (8a) precursor (with functional groups on the end of each arm) with PEG arms having a total MW of about 10,000 (10k) or 20,000 (20k) were used instead of the four-armed precursor.

A detailed procedure for making a hydrogel is as follows, using 4a20k SG as an example. Trilysine was mixed

into a 0.075 M Borate buffer at a concentration of 0.005 mg/ml. The resultant pH of the solution was approximately 10. 4a20k SG was reconstituted at 0.2 g/ml with a weak phosphate buffer at pH 4. The two liquid components were combined by forcing them through a static mixer into silicone tubing. The tubing was cut into disks and the gel was removed. Individual disks were weighed and placed into PBS at 37°C. After 24 hours the disks were weighed again and % swelling was calculated. Gel time was measured by injecting one component into a test tube containing the second component and a stir bar. A stopwatch was started at the time of injection and stopped when the stir bar exhibited a perceptible change in speed. The gels formed in the gel time measurement were used to determine persistence time. Individual gel plugs were placed in phosphate buffered saline at 37°C and monitored daily until they were not visible to the naked eye.

Other formulations were similarly made, with varying concentrations and pH: 8a15k SG (8 arm PEG, with the arms having a total combined MW of 15,000, terminated with succinimidyl glutrate) with 0.19 g PEG/ml phosphate, 0.012 g Trilysine/ml borate pH 10; 4a10k SS (4 arm PEG, with the arms having a total combined MW of 10,000, terminated with SS with 0.19 g PEG/ml phosphate, 0.008 g Trilysine/ml borate pH 10.

Table 1 shows the results obtained for these low swelling hydrogel formulations. Hydrogels prepared with ~ 9% solids and individual arm lengths less than about 2500 MW exhibited low swelling, compared to a hydrogel prepared from a precursor with individual arm lengths of about 5000, with other parameters being held essentially constant.

Table 1

| Formulation | Individual Ann Length /MW | Gel time ± std dev, s | Swelling ± std dev, % | Disappearance, days | Burst Strength, psi |
|---|---|---|---|---|---|
| 8a10k SG[i] | 1250 | 1.3 ± 0.04 | -32.7 ± 5.22 | 60 | 72 ± 11 |
| 6a10k SG[i] | 1667 | 1.5 ± 0.03 | -27.2 ± 2.54 | 60 | |
| 8a20k SG[i] | 2500 | 1.6 ± 0.11 | 12.3 ± 2.18 | 60 | |
| 4a20k SG[ii] | 5000 | 1.2 | 80 | 40 | 93 ± 36 |
| *i, n=3; ii, average based on multiple tests performed separately* | | | | | |

The materials shown in Table 1 were synthesized and tested to verify substitution levels over 95 %. Formulations were balanced at a 1:1 stoichiometry and pH was adjusted give similar gel times. All hydrogels had a gelation time of less than 5 seconds.

The 4 arm hydrogel swelled about 80% by weight (Table 1, 4a20k SG, with 4a indicating 4 arms, 20k indicating 20,000 MW PEG total for the arms, and SG indicating that each arm was terminated with succinimidyl glutarate). The 6 arm and 8 arm gels swelled only about 12% by weight or shrunk by about 27% or about 32% (Table 1).

Disappearance times were measured for the hydrogels (Table 1) by observing the gels in a clear plastic test tube and noting the time at which they were no longer visible to the naked eye, indicating that they were fully degraded. Burst strength was measured and found to be within acceptable ranges.

Example 2: Role of osmotic environment in swelling

The role of osmotic environment in swelling was tested by making a hydrogel using 4a20k SG as described in Example 1 and exposing it to a physiological buffered saline having a pH of 7.0-7.4 and an osmolarity of about 300 mOs or to a double-strength solution of the same saline. With n=3 (hydrogel plugs for each molarity of PBS), the swelling from gelation to equilibrium swelling (taken at 24 hours) averaged 68% for the physiological saline and 57% for double-strength saline. These results indicate that osmolarity differences inherent to the Swelling environment did not account for the reduced swelling of hydrogels formulated with precursors having different arm lengths because the changes in swelling were too small to account for the larger changes generally observed when the length of the arms was increased.

Example 3: Low-swelling hydrogels tested in vivo

Low-swelling hydrogels were implanted in the vertebral column in vivo. Formulation 1 was a hydrogel made from reacting a trilysine precursor reacted with 8a15k SG, with conditions effectively as described in Example 1 and Table 1. Precursors were applied using dual lumen applicators that mix the solutions and direct them to the site of application.

A total of 15 canines received full width laminectomies at both L2 and L5, after which a 1 cm midline durotomy was created, which was sutured closed. Animals were randomized to remain as controls (n=5 animals; no additional treatment prior to closure), or to receive formulation 1 application at both laminectomy sites using either a DUOFLO®

dual lumen applicator (Hemaedics Inc., Malibu, CA) (n=5 animals) or a MICROMYST™ dual lumen applicator (Confluent Surgical, Inc., Waltham, MA) (n=5 animals). Formulation 1 was observed to be adherent to the tissue, within a few seconds of its application.

The surgeries were preformed with a single midline skin incision (typically 15 cm length) made in all animals to gain access to both L2 and L5. Laminectomies (average 2.5 cm length, 1.3 cm width) were performed using standard or Kerrison rongeurs. All durotomies were midline and 1 cm in length, and all leaked CSF spontaneously following suturing.

Animals randomized to control had CSF weeping from suture holes at closure. All control sites (10/10) continued to weep CSF from the durotomy needle holes at the time of muscle and fascia closure.

All of the control animals developed postoperative subcutaneous fluid accumulations (5/5, 100%) within 1 to 3 days. All accumulations were contained by the sutured skin closure, and were present at the 1 week exam. Accumulations were presumed to be CSF, and were absorbed with flat incisions at the 4 week exam. Only 1 (10%) of the Formulation 1 animals exhibited postoperative subcutaneous fluid accumulation rate, compared to 5/5 (100%) of the controls. (Formula 1 applied with DUOFLO® had zero leaks, Formula 1 with MICROMYST™ had one leak, all controls leaked.) While not wishing to be bound by any theory the leak of Formula 1 was probably due to applicator (thickness of layer) and not formulation.

Animals randomized to receive Formulation 1 treatment underwent Valsalva's Maneuver to 20 cm $H_2O$ following application. No sites treated with Formulation 1 (20/20) had leakage following hydrogel application, despite the Valsalva's Maneuver.

The average volume applied and thickness over the suture line for the MICROMYST™ group was 1.3 ml volume and 2.7 mm thickness, with 2.2 ml volume and 3.3 mm and thickness for the DUOFLO® group. Since the laminectomy width was the full width of the dural sac, the gutters in each laminectomy site were deep and extended down to the nerve roots. Therefore, while the average Formulation 1 thickness over the sutures was about 3.3 mm, the thickness due to runoff in the gutters was probably approaching 8-10 mm in some cases.

All animals were evaluated for neurological deficits at 1, 4, 8 and 16 weeks. Assessments focused on neurological sequelae for alertness, motor function, cranial nerve function and posture. No neurological deficits were noted in any of the animals enrolled. With the exception of one early death for causes unrelated to the surgeries, all animals remained healthy with no detectable sequelae from the initial surgical procedure. These results indicate that low-swelling hydrogels were effective as applied to the tissue inside the vertebral column and substantially exterior to a theca in the vertebral column, including peridural and epidural spaces and spinal nerves or nerve roots near the vertebral column.

## Claims

1. A composition comprising:

   a first synthetic precursor possessing first functional groups and having a molecular weight of from 100 to 1500 Da,
   a second synthetic precursor comprising a multi-armed precursor possessing a core and having 6 arms or 8 arms, the arms each comprising a molecular weight from 1250 to 2500 Da and possessing second functional groups at the ends thereof;
   wherein the first functional groups crosslink with the second functional groups thereby forming a biodegradable hydrogel the hydrogel swells from -50% to 50%
   for use in the treatment of tissue inside the vertebral column of a patient.

2. The composition according to claim 1-, wherein the hydrogel shrinks by a weight decrease of from 1 % to 50%.

3. The composition according to claim 1 or claim 2, wherein the first functional groups comprise nucleophiles and the second functional groups comprise electrophiles.

4. The composition according to any preceding claim, wherein the first synthetic precursor is selected from the group consisting of dilysines, trilysines, and tetralysines and the multifunctional centre of the second synthetic precursor is selected from the group consisting of polyethers, polyamine acids, proteins, and polyols

5. The composition according to any preceding claim, further comprising a drug.

6. The composition according to any preceding claim further comprising a visualization agent.

7. The composition according to claim 6, wherein the visualization agent comprises a dye selected from the group

consisting of FD&C Blue #1, FD&C Blue #2, FD&C Blue #3, D&C Green #6, methylene blue, and combinations thereof.

**8.** The composition according to any preceding claim wherein the hydrogel is crosslinked in less than 10 seconds after mixing the first synthetic precursor and second synthetic precursor with each other.

**9.** The composition according to any preceding claim, wherein the first synthetic precursor is selected from the group consisting of dilysines, trilysines, and tetralysines.

**10.** The composition according to any preceding claim, wherein the first synthetic precursor comprises an oligopeptide sequence of no more than five residues comprising at least two lysine groups.

**11.** The composition according to any preceding claim, wherein the core is selected from the group consisting of polyethers, polyamine acids, proteins, and polyols.

**12.** The composition according to any preceding claim, wherein the core_is selected from the group consisting of polyethylene glycol, polyethylene oxide, polyethylene oxide-copolypropylene oxide, co-polyethylene oxide copolymers, polyvinyl alcohol, polyvinyl pyrrolidinone, poly(amino acids), dextran, proteins, derivatives thereof, and combinations thereof.

**13.** A composition according to any preceding claim, which is biodegradable.

**Patentansprüche**

**1.** Zusammensetzung, die umfasst:

ein erstes synthetisches Vorläuferprodukt, das erste funktionale Gruppen besitzt und ein Molekulargewicht von 100 bis 1.500 Da aufweist,
ein zweites synthetisches Vorläuferprodukt, das ein mehrarmiges Vorläuferprodukt umfasst, das einen Kern besitzt und 6 Arme oder 8 Arme aufweist, wobei jeder Arm ein Molekulargewicht von 1.250 bis 2.500 Da umfasst und zweite funktionale Gruppen an dem Ende desselben besitzt;
wobei die ersten funktionalen Gruppen mit den zweiten funktionalen Gruppen vernetzen, wodurch ein bioabbaubares Hydrogel gebildet wird, wobei das Hydrogel um -50 % bis 50 % quillt
für eine Verwendung bei der Behandlung von Gewebe innerhalb der Wirbelsäule eines Patienten.

**2.** Zusammensetzung nach Anspruch 1, wobei das Hydrogel durch eine Gewichtsabnahme um 1% bis 50 % schrumpft.

**3.** Zusammensetzung nach Anspruch 1 oder 2, wobei die ersten funktionalen Gruppen Nucleophile umfassen und die zweiten funktionalen Gruppen Electrophile umfassen.

**4.** Zusammensetzung nach einem vorhergehenden Anspruch, wobei das erste synthetische Vorläuferprodukt aus der Gruppe ausgewählt ist, die aus Dilysinen, Trilysinen und Tetralysinen besteht, und wobei das multifunktionale Zentrum des zweiten synthetischen Vorläuferprodukts aus der Gruppe ausgewählt ist, die aus Polyethern, Polyaminsäuren, Proteinen und Polyolen besteht.

**5.** Zusammensetzung nach einem vorhergehenden Anspruch, die ferner ein Arzneimittel umfasst.

**6.** Zusammensetzung nach einem vorhergehenden Anspruch, die ferner ein bildgebendes Mittel umfasst.

**7.** Zusammensetzung nach Anspruch 6, wobei das bildgebende Mittel einen Farbstoff umfasst, der aus der Gruppe ausgewählt ist, die aus FD&C Blau #1, FD&C Blau #2, FD&C Blau #3, D&C Grün #6, Methylenblau und Kombinationen davon besteht.

**8.** Zusammensetzung nach einem vorhergehenden Anspruch, wobei das Hydrogel in weniger als 10 Sekunden vernetzt wird, nachdem das erste synthetische Vorläuferprodukt und das zweite synthetische Vorläuferprodukt miteinander gemischt worden sind.

**9.** Zusammensetzung nach einem vorhergehenden Anspruch, wobei das erste synthetische Vorläuferprodukt aus der

Gruppe ausgewählt ist, die aus Dilysinen, Trilysinen und Tetralysinen besteht.

10. Zusammensetzung nach einem vorhergehenden Anspruch, wobei das erste synthetische Vorläuferprodukt eine Oligopeptidsequenz von nicht mehr als fünf Resten, die mindestens zwei Lysingruppen umfassen, umfasst.

11. Zusammensetzung nach einem vorhergehenden Anspruch, wobei der Kern aus der Gruppe ausgewählt ist, die aus Polyethern, Polyaminsäuren, Proteinen und Polyolen besteht.

12. Zusammensetzung nach einem vorhergehenden Anspruch, wobei der Kern aus der Gruppe ausgewählt ist, die aus Polyethylenglycol, Polyethylenoxid, Polyethylenoxidcopolypropylenoxid, Copolyethylenoxidcopolymeren, Polyvinylalkohol, Polyvinylpyrrolidinon, Poly(aminosäuren), Dextran, Proteinen, aus Derivaten derselben und aus Kombinationen derselben besteht.

13. Zusammensetzung nach einem vorhergehenden Anspruch, die bioabbaubar ist.

**Revendications**

1. Composition comprenant :

   un premier précurseur synthétique possédant des premiers groupes fonctionnels et ayant une masse moléculaire de 100 à 1 500 Da,
   un second précurseur synthétique comprenant un précurseur à bras muliples possédant un coeur et ayant 6 bras ou 8 bras, les bras ayant chacun une masse moléculaire de 1 250 à 2 500 Da et possédant de seconds groupes fonctionnels aux extrémités de ceux-ci ;
   dans laquelle les premiers groupes fonctionnels se réticulent avec les seconds groupes fonctionnels formant de ce fait un hydrogel biodégradable, l'hydrogel gonflant de -50 % à 50 %
   à utiliser dans le traitement de tissu à l'intérieur de la colonne vertébrale d'un patient.

2. Composition selon la revendication 1, dans laquelle l'hydrogel se contracte par une diminution de masse de 1 % à 50 %.

3. Composition selon la revendication 1 ou la revendication 2, dans laquelle les premiers groupes fonctionnels comprennent des nucléophiles et les seconds groupes fonctionnels comprennent des électrophiles.

4. Composition selon l'une quelconque des revendications précédentes, dans laquelle le premier précurseur synthétique est sélectionné à partir du groupe constitué par des dilysines, des trilysines et des tétralysines et le centre multifonctionnel du second précurseur synthétique est sélectionné à partir du groupe constitué par des polyéthers, des acides de polyamine, des protéines et des polyols.

5. Composition selon l'une quelconque des revendications précédentes, comprenant en outre un médicament.

6. Composition selon l'une quelconque des revendications précédentes, comprenant en outre un agent de visualisation.

7. Composition selon la revendication 6, dans laquelle l'agent de visualisation comprend un colorant sélectionné à partir du groupe constitué par FD&C Bleu n° 1, FD&C Bleu n° 2, FD&C Bleu n° 3, D&C Vert n° 6, bleu de méthylène et des combinaisons de ceux-ci.

8. Composition selon l'une quelconque des revendications précédentes, dans laquelle l'hydrogel est réticulé en moins de 10 secondes après mélange du premier précurseur synthétique et du second précurseur synthétique l'un avec l'autre.

9. Composition selon l'une quelconque des revendications précédentes, dans laquelle le premier précurseur synthétique est sélectionné à partir du groupe constitué par des dilysines, des trilysines et des tétralysines.

10. Composition selon l'une quelconque des revendications précédentes, dans laquelle le premier précurseur synthétique comprend une séquence d'oligopeptides de pas plus de cinq résidus comprenant au moins deux groupes lysine.

**11.** Composition selon l'une quelconque des revendications précédentes, dans laquelle le coeur est sélectionné à partir du groupe constitué par des polyéthers, des acides de polyamine, des protéines et des polyols.

**12.** Composition selon l'une quelconque des revendications précédentes, dans laquelle le coeur est sélectionné à partir du groupe constitué par du polyéthylène glycol, de l'oxyde de polyéthylène, de l'oxyde de polyéthylène - oxyde de copolypropylène, des copolymères d'oxyde de copolyéthylène, de l'alcool de polyvinyle, du polyvinyle pyrrolidinone, des acides poly(aminés), du dextrane, des protéines, des dérivés de ceux-ci et des combinaisons de ceux-ci.

**13.** Composition selon l'une quelconque des revendications précédentes, qui est biodégradable.

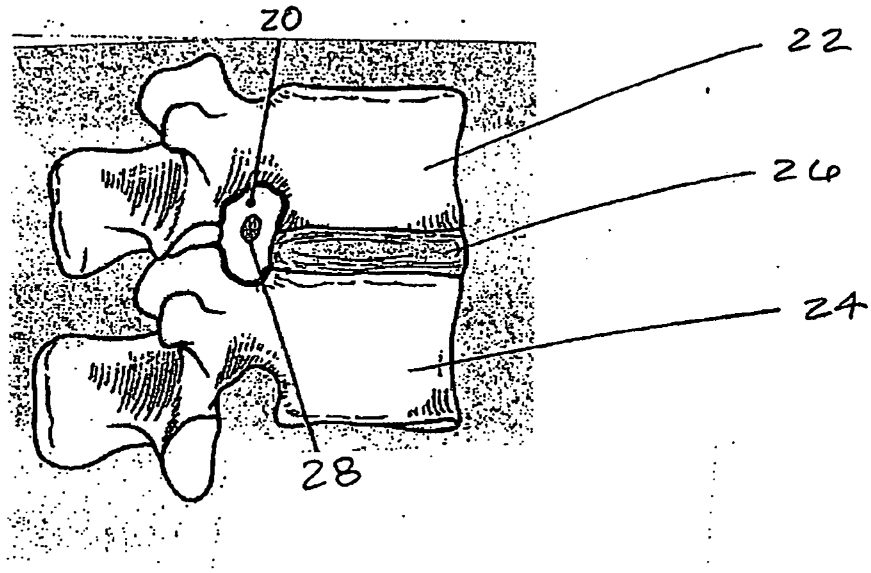


FIG 1

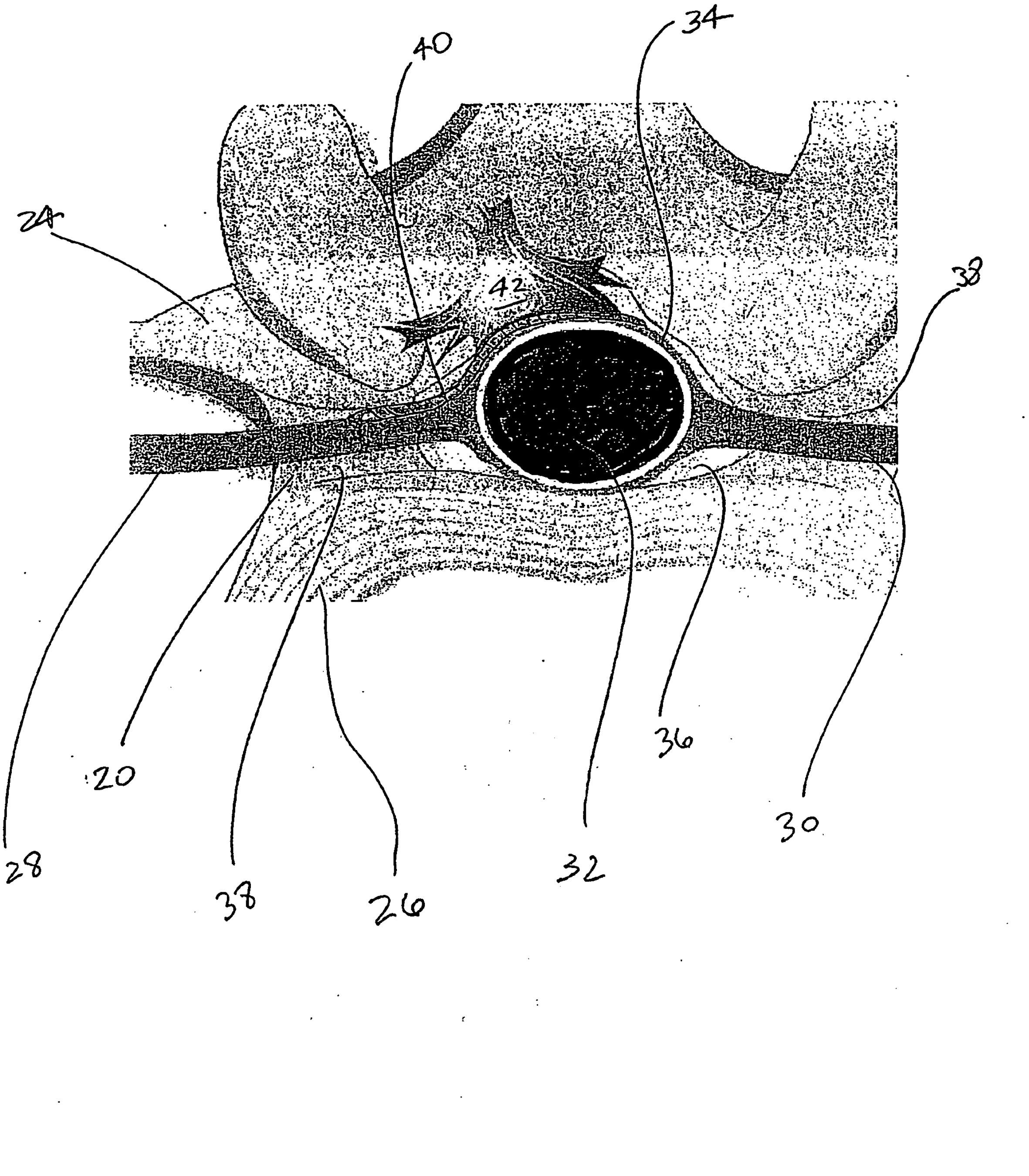


FIG 2.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2003012734 A
- US 6656200 B
- US 5874500 B
- US 5543441 B
- US 5514379 B
- US 5410016 B
- US 5162430 B
- US 5324775 B
- US 5752974 B
- US 5550187 B
- US 4938763 A, Dunn
- US 5100992 A
- US 4826945 A, Cohn
- US 4741872 A
- US 5160745 A, De Luca
- US 5410016 A, Hubbell
- US 4874368 A
- US 4631055 A
- US 4735616 A
- US 4359049 A
- US 4978336 A
- US 5116315 A
- US 4902281 A
- US 4932942 A
- US 6179862 A
- US 6673093 A
- US 6152943 A
- US 7009034 B